# EUROPÄISCHE PATENTSCHRIFT

(11) **EP 4 147 671 B1**
(45) Veröffentlichungstag und Bekanntmachung des Hinweises auf die Patenterteilung: **15.05.2024**
(21) Anmeldenummer: 22194191.7
(22) Anmeldetag: 06.09.2022
(51) Int. Cl.: A61C 19/00, A61C 8/00, A61B 50/30, G01M 3/40

(54) **DENTALVORRICHTUNGSVERPACKUNG UND VERPACKUNGSSYSTEM**
DENTAL DEVICE PACKAGING AND PACKAGING SYSTEM
EMBALLAGE DE DISPOSITIF DENTAIRE ET SYSTÈME D'EMBALLAGE

(30) Priorität: 08.09.2021 DE 102021123227
(43) Veröffentlichungstag der Anmeldung: 15.03.2023
(73) Patentinhaber: medentis medical GmbH, 53474 Bad Neuenahr-Ahrweiler (DE)
(72) Erfinder: Knieps, Torsten, 53505 Berg (DE)
(74) Vertreter: Müller Schupfner & Partner Patent- und Rechtsanwaltspartnerschaft mbB (Muc)

(56) Entgegenhaltungen:
- WO-A1-2021/062381
- US-A1- 2020 363 363

## Beschreibung

Die Erfindung betrifft eine Sterilgutverpackung, welche eine Dentalvorrichtungsverpackung ist, und ein Verpackungssystem.

Dentalvorrichtungen - als Beispiel eines Sterilguts - sind bereits aus dem Stand der Technik bekannt. Diese dienen dazu, im Dentalbereich eines Patienten eingesetzt zu werden, beispielsweise als ein Werkzeug oder als eine Vorrichtung, welche im Dentalraum des Patienten verbleibt. Um eine Infektion im Dentalbereich des Patienten zu verhindern oder zumindest deren Risiko zu minimieren, müssen diese Dentalvorrichtungen steril verpackt werden. Es kommt jedoch häufig vor, dass die Verpackungen, z.B. durch Risse, ihre Fähigkeit verlieren, die Dentalvorrichtung in einer sterilen Umgebung zu verwahren.

Die WO 2021/062381 A1 zeigt einen Behälter mit einem Innenvolumen, das zur Aufnahme eines elektrisch leitfähigen Produkts konfiguriert ist. Der Behälter umfasst eine Wand mit einer Innenschicht, einer Außenschicht und einer Zwischenschicht zwischen der Innenschicht und der Außenschicht. Die Innenschicht besteht aus einem elektrisch isolierenden Material und die Zwischenschicht besteht aus einem elektrisch leitenden Material.

Es ist daher die Aufgabe der vorliegenden Erfindung eine Verpackung für ein Sterilgut, nämlich eine Dentalvorrichtung, bereitzustellen, welche das Erkennen des Integritätsverlustes der Verpackung vereinfacht, um so die Gesundheit des Patienten zu fördern.

Diese Aufgabe wird mit einer Sterilgutverpackung gemäß dem Anspruch 1, mit einer Verwendung einer Sterilgutverpackung gemäß Anspruch 13, einem Verfahren zur Herstellung einer Sterilgutverpackung gemäß Anspruch 14 und mit einem Verpackungssystem gemäß dem Anspruch 15 gelöst. Weitere Vorteile, Merkmale und Ausführungsformen ergeben sich aus den abhängigen Ansprüchen, der Beschreibung sowie aus den Figuren.

Erfindungsgemäß ist eine Sterilgutverpackung, nämlich eine Dentalvorrichtungsverpackung. Vorteilhafterweise umfasst die Sterilgutverpackung eine Verpackungsfolie, wobei die Sterilgutverpackung einen Innenraum aufweist, wobei die Verpackungsfolie den Innenraum der Sterilgutverpackung gegenüber der Umgebung zumindest teilweise abgrenzt, wobei die Verpackungsfolie eine Trennschicht aufweist, wobei die Verpackungsfolie eine erste Leitschicht und eine zweite Leitschicht aufweist, wobei die erste und die zweite Leitschicht jeweils elektrisch leitend sind und jeweils einen Primäranschluss aufweisen, wobei die Trennschicht zwischen der ersten Leitschicht und der zweiten Leitschicht angeordnet ist. Die Sterilgutverpackung dient dazu, Sterilgüter gegenüber äußeren Einflüssen sicher zu verwahren bzw. zu verpacken und vorteilhafterweise deren Kontamination mit Bakterien zu verhindern. Sterilgüter sind insbesondere Güter, Systeme, Vorrichtungen oder Instrumente, welche in einen keimfreien und sterilen Zustand gebracht wurden, insbesondere dienen die Sterilgüter dazu, temporär oder dauerhaft in den Körper eines Menschens einzudringen und/oder in diesem angeordnet zu werden. Vorteilhafterweise ist eine Dentalvorrichtung ein Sterilgut. Die Dentalvorrichtungsverpackung dient dazu, Dentalvorrichtungen gegenüber äußeren Einflüssen sicher zu verwahren bzw. zu verpacken. Dentalvorrichtungen sind dabei insbesondere dentalmedizische und zahnmedizinische Apparate und Instrumente, z.B. Dentalbohrer, Dentalsonden, Dentalnadeln, Dentalfarbenführer, Dentalartikulatoren, Dentalbohrereinsätze, medizinische Geräte für Dentalhygieniker, Dentalvorrichtungen zur Behandlung von obstruktiver Schlafapnoe, Dentalsonden zur Verwendung für Zahnbehandlungen, Geräte für die Wiedergabe dentaler Bilder für medizinische Zwecke, sowie Prothesen, Aufbauten und dentale Implantate sowie deren Implantationswerkzeuge und Zubehör, insbesondere Zahnersatz, Zahnprothesen und Aufbauten sowie Montagewerkzeuge für Zahnimplantate wie z.B. Detaldrehmomentschlüssel oder Schraubendreher. In anderen Worten können Dentalvorrichtungen dadurch gekennzeichnet sein, dass deren temporärer oder dauerhafter Verbleib im Mundraum bei deren Benutzung vorgesehen ist. Die Sterilgutverpackung, nämlich die Dentalvorrichtungsverpackung, umfasst zumindest eine Verpackungsfolie. Unter einer Folie ist insbesondre eine flächige Ausgestaltung zu verstehen, welche eine gewisse Materialstärke aufweist, und vorteilhafterweise unter mechanischen Einflüssen verbogen werden kann. Eine derartige Verpackungsfolie kann insbesondere eine Materialstärke aufweisen, welche unter 1 mm, bevorzugt unter 500 µm, bevorzugt unter 300 µm und besonders stark bevorzugt unter 100 µm, liegt. Die Verpackungsfolie ist vorteilhafterweise durchsichtig, um einen Einblick in den Innenraum der Sterilgutverpackung, nämlich der Dentalvorrichtungsverpackung, zu ermöglichen. Der Innenraum der Sterilgutverpackung ist insbesondere derjenige Raum, welcher dazu ausgelegt ist, ein Sterilgut, nämlich eine Dentalvorrichtung, aufzunehmen. Um den Innenraum gegenüber der Umgebung und somit gegenüber Umwelteinflüssen zu schützen, ist der Innenraum der Dentalvorrichtungsverpackung, zumindest teilweise durch die Verpackungsfolie gegenüber der Umgebung abgegrenzt. Unter einem Abgrenzen ist dabei insbesondere zu verstehen, dass durch die Verpackungsfolie Gegenstände und/oder Gase und/oder Bakterien von der Umgebung zumindest teilweise daran gehindert sind, in den Innenraum der Dentalvorrichtungsverpackung, einzudringen. Die Verpackungsfolie ist insbesondere mehrschichtig aufgebaut. Einer dieser Schichten der Verpackungsfolie kann eine Trennschicht sein. Die Trennschicht dient dabei dazu, zwei andere Schichten voneinander direkt oder indirekt zu trennen. In anderen Worten dient die Trennschicht dazu, eine Separation zwischen anderen Schichten der Verpackungsfolie auszuführen bzw. bereitzustellen. Unter einer Schicht im Sinne der Erfindung sind insbesondere lediglich flächige Elemente zu verstehen. Daher sind insbesondere z.B. Drähte oder ähnliche Elemente vorteilhafter Weise keine Schichten im Sinne der Erfindung. Diese anderen Schichten können dabei direkt oder indirekt flächig mit der Trennschicht verbunden sein. Vorteilhafterweise dient die Trennschicht auch dazu, die mechanische Stabilität der Verpackungsfolien zu erreichen bzw. bereitzustellen. Eine derartige mechanische Stabilität durch die Trennschicht kann insbesondere dann erreicht werden, wenn zumindest 30 %, bevorzugt zumindest 50 % und besonders bevorzugt zumindest 70 % der Verpackungsfoliendicke bzw. Verpackungsfolienmaterialstärke durch die Trennschicht ausgebildet sind bzw. ist. Maßgeblich ist dabei insbesondere die gemittelte Materialstärke der Verpackungsfolie bzw. der Trennschicht über die gesamte Erstreckungsbreite und Länge der Verpackungsfolie. Neben der Trennschicht weist die Verpackungsfolie auch eine erste Leitschicht und eine zweite Leitschicht auf. Diese erste Leitschicht und die zweite Leitschicht sind elektrisch leitend, sodass prinzipiell ein elektrischer Strom durch die erste und/oder durch die zweite Leitschicht fließen kann. Um jeweils eine Einleitungsmöglichkeit für einen elektrischen Strom und/oder eine elektrische Spannung in die erste bzw. zweite Leitschicht zu ermöglichen, verfügen diese Schichten jeweils über einen Primäranschluss. In anderen Worten können der Primäranschluss der ersten Leitschicht und der Primäranschluss der zweiten Leitschicht dazu verwendet werden, die jeweilige Leitschicht mit einer Strom- oder Spannungsquelle zu verbinden. Zwischen der ersten Leitschicht und der zweiten Leitschicht ist die Trennschicht angeordnet. Unter einem "zwischen" ist insbesondere zu verstehen, dass - in Dickenrichtung der Verpackungsfolie - auf der einen Seite der Trennschicht die erste Leitschicht angeordnet ist und auf der gegenüberliegenden Seite der Trennschicht - in Dickenrichtung - die zweite Leitschicht. Durch das Vorsehen von zwei Leitschichten, welche jeweils auf unterschiedlichen Seiten der Trennschicht angeordnet sind, kann ein Durchstoßen der Trennschicht in einfacher Weise detektiert werden, indem eine Strom- bzw. Spannungsquelle mit einem ersten Pol an den Primäranschluss der ersten Leitschicht und mit einem bzw. dessen zweiten Pol an den Primäranschluss der zweiten Leitschicht angelegt wird. Sollte in einem derartigen Fall ein Strom zwischen der ersten Leitschicht und der zweiten Leitschicht flie-ßen und/oder einen Grenzwert überschreiten, so kann eine Versagung der Integrität der Verpackungsfolie bzw. der Trennschicht in eindeutiger Weise festgestellt werden oder zumindest indiziert werden. Somit erreicht die Sterilgutverpackung den Effekt, dass hierdurch in einfacher Weise ein Erkennen des Integritätsverlusts der Sterilgutverpackung nämlich der Dentalvorrichtungsverpackung detektiert oder zumindest indiziert werden kann.

Vorteilhafterweise ist die Trennschicht dicker als die erste und/oder als die zweite Leitschicht. Hierdurch kann erreicht werden, dass besonders kostengünstig eine hohe Festigkeit erreicht wird.

Vorteilhafterweise bedecken die erste und/oder die zweite Leitschicht flächig die Trennschicht. Unter einem flächigen Bedecken ist insbesondere zu verstehen, dass zumindest 60 %, bevorzugt zumindest 75 %, besonders bevorzugt zumindest 85 % und besonders stark bevorzugt zumindest 95 %, der der Umgebung oder der dem Innenraum zugewandten Fläche der Trennschicht direkt oder indirekt von der ersten und/oder der zweiten Leitschicht bedeckt sind. Durch diese großflächige Bedeckung der Trennschicht durch die erste und/oder durch die zweite Leitschicht kann in besonders sicherer Weise ein großer Teil der Verpackungsfolie in einfacher Weise in Hinblick auf deren Integrität untersucht bzw. analysiert werden. Daher kann durch eine derartige Ausgestaltung eine besonders sichere Detektion bzw. Indikation im Hinblick auf einen Integritätsverlust der Sterilgutverpackung erreicht werden.

Zweckmäßigerweise ist die Trennschicht nicht elektrisch leitend. Durch die Ausbildung der Trennschicht dahingehend, dass diese einen vollständigen Isolator darstellt, welche eine elektrische Trennung zwischen der ersten Leitschicht und der zweiten Leitschicht bewerkstelligt, kann in besonders sicherer Weise festgestellt werden, ob ein Integritätsverlust der Sterilgutverpackung eingetreten ist oder nicht. Alternativ bevorzugt kann die Trennschicht jedoch auch aus einem hochohmigen Material ausgebildet sein und/oder mit einem Isolatormaterial beschichtet sein. Vorteilhafterweise ist die Trennschicht bakterienundurchlässig. Durch das Ausbilden der Trennschicht dahingehend, dass diese bakterienundurchlässig ist, kann eine besonders gute Abschirmung des Innenraums der Sterilgutverpackung, insbesondere für sterilisierte Dentalvorrichtungen, erreicht werden. Um eine derartige bakterielle Undurchlässigkeit zu erreichen ist es vorteilhaft, wenn die Porengröße der Trennschicht < 0,5 µm, bevorzugt < 0,3 µm, und besonders bevorzugt < 0,2 µm ist. Unter einer Bakterienundurchlässigkeit ist dabei insbesondere zu verstehen, dass Bakterien von der einen Seite der Trennschicht nicht auf die andere Seite der Trennschicht durch die Trennschicht gelangen können.

Vorteilhafterweise ist die Trennschicht aus Kunststoff, insbesondere aus PET, PETG und/oder PE-HD und/oder PU, insbesondere TPU. Durch die Ausbildung der Trennschicht dahingehend, dass diese aus einem Kunststoff ausgebildet ist und/oder einen Kunststoff umfasst, kann eine besonders kostengünstige Ausgestaltung der Trennschicht erreicht werden. Sollte die Trennschicht aus PETG hergestellt sein bzw. bestehen, so kann hierdurch eine besonders gute Möglichkeit einer formstabilen Ausgestaltung der Verpackungsfolie erreicht werden. In anderen Worten kann daher durch eine Verwendung von PETG als Material der Trennschicht erzielt werden, dass die mögliche körperliche Gestalt der Trennschicht, beispielsweise durch Tiefziehen, definiert ausgebildet werden kann. Sollte die Trennschicht hingegen aus PE-HD (Polyethylen hoher Dichte) ausgebildet sein, so kann hierdurch eine besonders sterile Möglichkeit der Abgrenzung des Innenraums der Sterilgutverpackung gegenüber der Umgebung erreicht werden, insbesondere wenn eine forminstabile Ausgestaltung der Trennschicht gewünscht ist. Eine derartige forminstabile Ausgestaltung der Trennschicht kann insbesondere dann wünschenswert sein, wenn die Verpackungsfolie als Verschluss einer Öffnung dienen soll. Ein derartiger Verschluss kann insbesondere über eine stoffschlüssige Festlegung mit den die Öffnung ausbildenden Bestandteilen der Sterilgutverpackung, nämlich der Dentalvorrichtungsverpackung, erfolgen.

Diese öffnungsausbildenden Elemente bzw. Bestandteile der Sterilgutverpackung können dabei ebenfalls durch eine und/oder durch eine Vielzahl von Verpackungsfolien wie vorgehend und nachfolgend beschrieben ausgebildet sein. In anderen Worten können die Abdeckung und/oder der öffnungsausbildende Teil der Sterilgutverpackung eine wie vorgehend und nachfolgend beschriebene Verpackungsfolie aufweisen und/oder jeweils durch eine derartige ausgebildet sein. Hierdurch kann die Integritätsüberwachung der Sterilgutverpackung bzw. die Integritätssensierung der Sterilgutverpackung erweitert bzw. verbessert werden.

Zweckmäßigerweise ist der Primäranschluss der ersten Leitschicht mit einem Pol einer elektrischen Energiequelle verbunden und/oder verbindbar, und/oder wobei der Primäranschluss der zweiten Leitschicht mit dem anderen Pol der elektrischen Energiequelle verbunden ist oder verbindbar ist. Hierdurch kann in besonders einfacher Weise, insbesondere durch eine Kapazitätsmessung oder eine Spannungsabfalldetektion und/oder das Messen einer Stromstärke und/oder eines Wiederstands festgestellt werden, ob ein Strom vom Primäranschluss der ersten Leitschicht zu dem Primäranschluss der zweiten Leitschicht und/oder umgekehrt fließen kann. Die elektrische Energiequelle kann dabei insbesondere eine Gleichstrom- oder eine Wechselstromenergiequelle sein. Vorteilhafterweise ist die elektrische Energiequelle dabei eine Spannungsquelle oder eine Stromquelle. Die Pole der elektrischen Energiequelle können insbesondere die Plus- bzw. die jeweiligen Minuspole der elektrischen Energiequelle sein.

Vorteilhafterweise ist die elektrische Energiequelle eine Wechselstromquelle und/oder eine Gleichstromquelle. Bei der Verwendung einer Gleichstromquelle, insbesondere in Form einer Batterie, kann eine besonders kostengünstige und leicht zu transportierende Sterilgutverpackung, nämlich Dentalvorrichtungsverpackung, erreicht werden. Die Batterie kann dabei vorteilhafterweise ein Blei- oder ein Lithium-Ionen-Akkumulator sein. Sollte die elektrische Energiequelle jedoch eine Wechselstromquelle darstellen, so kann hierdurch, insbesondere durch eine kapazitive Messung, eine Integritätsprüfung der ersten Leitschicht und/oder der zweiten Leitschicht und/oder der Trennschicht erfolgen, sodass durch eine derartige Ausgestaltung nicht nur ein Integritätsverlust der Trennschicht ermittelt werden kann, sondern auch Integritätsverluste der ersten bzw. zweiten Leitschicht. Besonders bevorzugt ist es, wenn die elektrische Energiequelle eine umschaltbare elektrische Energiequelle ist, welche sowohl als eine Wechselstromquelle als auch als eine Gleichstromquelle agieren kann. Unter einer Wechselstromquelle und einer Gleichstromquelle ist insbesondere nicht nur eine Stromquelle sondern auch eine Spannungsquelle zu verstehen.

Zweckmäßigerweise weisen die erste Leitschicht und/oder die zweite Leitschicht einen Sekundäranschluss auf und/oder sind jeweils mit einem Sekundäranschluss verbunden. Durch das Vorsehen eines Primär- und eines Sekundäranschlusses an der ersten Leitschicht und/oder der zweiten Leitschicht kann in einfacher Weise ein Wiederstand zwischen dem Primär- und dem Sekundäranschluss ermittelt werden, um so die Integrität der ersten und/oder der zweiten Leitschicht zu ermitteln. In anderen Worten kann die Widerstandsbestimmung zwischen dem Primäranschluss der ersten Leitschicht und dem Sekundäranschluss der ersten Leitschicht bzw. dem Primäranschluss der zweiten Leitschicht und dem Sekundäranschluss der zweiten Leitschicht ein Rückschluss auf die Dicke und die Unversehrtheit der ersten Leitschicht bzw. der zweiten Leitschicht erlauben. Somit kann durch eine derartige Ausgestaltung eine Funktionsüberprüfung der Integritätssensierung durch die erfindungsgemäße Sterilgutverpackung erfolgen.

Vorteilhafterweise ist die Trennschicht, die erste Leitschicht und/oder die zweite Leitschicht gasdurchlässig und/oder gammastrahlungsdurchlässig. Durch die Ausbildung dahingehend, dass die Trennschicht, die erste Leitschicht und/oder die zweite Leitschicht gasdurchlässig ist, ermöglicht die Sterilgutverpackung eine Gas- bzw. wasserdampfbasierte Sterilisation einer in der Sterilgutverpackung vorhandenen bzw. in den Innenraum der Dentalverpackungsvorrichtung einbringbares Sterilgut, in Form einer Dentalvorrichtung. Eine derartige Gasdurchlässigkeit kann insbesondere dadurch erreicht werden, dass die Trennschicht, die erste Leitschicht und/oder die zweite Leitschicht eine Porengröße im Bereich von 1 - 50 µm aufweisen. Alternativ oder zusätzlich bevorzugt können die Trennschicht, die erste Leitschicht und/oder die zweite Leitschicht auch gammastrahlungsdurchlässig sein, um so den Innenraum bzw. das in dem Innenraum vorhandene Sterilgut in Form einer Dentalvorrichtung, zu sterilisieren, insbesondere durch eine Gammabestrahlung. In anderen Worten kann die Erfindung auch ein Verfahren zur Sterilisation eines Sterilguts in einer Sterilgutverpackung wie vorgehend und nachfolgend betreffen. Insbesondere umfasst ein derartiges Verfahren dabei die Schritte der Bereitstellung einer Sterilgutverpackung mit einer Dentalvorrichtung, welche im Innenraum der Sterilgutverpackung angeordnet ist, wobei die Sterilgutverpackung die vorgehend und nachfolgend beschriebenen Merkmale aufweisen kann, und Einbringen eines sterilisierenden Gases oder von Wasserdampf durch die Trennschicht, die erste Leitschicht und/oder die zweite Leitschicht in den Innenraum der Sterilgutverpackung und/oder Bestrahlen des Innenraums der Sterilgutverpackung mit Gammastrahlung. Durch ein derartiges Verfahren kann insbesondere eine Sterilisation eines Sterilguts innerhalb der Sterilgutverpackung erreicht werden.

Zweckmäßigerweise ist die erste und/oder die zweite Leitschicht zumindest teilweise, bevorzugt vollständig, aus Graphit ausgebildet. Durch eine derartige Ausgestaltung kann eine besonders kostengünstige und dennoch leitfähige erste und/oder zweite Leitschicht erreicht werden. Zweckmäßigerweise kann dieses Graphit dabei durch Aufdampfung aufgebracht sein, insbesondere durch Aufdampfung auf die Trennschicht.

Zweckmäßigerweise ist die erste und/oder die zweite Leitschicht zumindest teilweise, bevorzugt vollständig, aus einem Material ausgebildet und/oder ist die erste und/oder die zweite Leitschicht zumindest teilweise, bevorzugt vollständig, mit einem Material beschichtet, wobei das Material derart ausgebildet ist, dass dieses seine Farbe bei einem durch die erste und/oder die zweite Leitschicht strömenden elektrischen Strom ändert. Hierdurch kann in besonders einfacher und schneller Weise eine optische Identifikation eines Integritätsverlustes der Sterilgutverpackung festgestellt werden. Ein Material, welches durch einen Stromfluss seine Farbe ändert ist insbesondere Wolframtrioxid oder Polyanilin.

Vorteilhafterweise liegt das Verhältnis der Dicke der ersten Leitschicht bzw. der Dicke der zweiten Leitschicht zu der Dicke der Trennschicht in einem Bereich von 0,25 bis 1,5, bevorzugt in einem Bereich von 0,4 bis 1 und besonders bevorzugt in einem Bereich von 0,45 bis 0,55. Die Dicke der ersten Leitschicht, der zweiten Leitschicht und/oder der Trennschicht bestimmt sich insbesondere in Dickenrichtung der jeweiligen Schicht. Diese Dickenrichtung steht insbesondere senkrecht auf den beiden anderen Richtungen, in welche die jeweilige Schicht ihre größten Hauptdimensionen aufweisen. Diese beiden Richtungen der größten Hauptdimensionen stehen insbesondere ebenfalls senkrecht zueinander. Alternativ oder zusätzlich bevorzugt ist die Dickenrichtung die lokale Normalenrichtung der jeweiligen Schicht. Sollte das Verhältnis der Dicke der ersten Leitschicht zu der Dicke der Trennschicht bzw. die Dicke der zweiten Leitschicht zu der Dicke der Trennschicht in einem Bereich von 0,25 bis 1,5 liegen, so kann hierdurch eine besonders einfache und kostengünstige Herstellung der Verpackungsfolie erreicht werden. Um eine besonders einfache Integritätsprüfung zu erreichen, insbesondere bei einer forminstabilen Ausgestaltung der Verpackungsfolie, sollte das Verhältnis in einem Bereich von 0,4 bis 1 liegen. Um eine besonders mechanisch belastbare Ausgestaltung der Festlegung der ersten Leitschicht bzw. der zweiten Leitschicht in Relation zu der Trennschicht zu erreichen, sollte das Verhältnis insbesondere in einem Bereich von 0,45 bis 0,55 liegen.

Vorteilhafterweise ist die Verpackungsfolie formstabil oder forminstabil. Unter einem formstabil ist dabei zu verstehen, dass die äußere Ausgestaltung der Verpackungsfolie ihre Form trotz einfacher mechanischer Belastung stabil beibehält. In anderen Worten ist eine Formstabilität insbesondere dann gegeben, wenn die äu-ßere Ausgestaltung bzw. die globale Ausgestaltung der Verpackungsfolie beispielsweise durch die Schwerkraft ihre Ausgestaltung nicht verändert und/oder nur geringfügig verändert wird. Somit sind beispielsweise Balken oder Bretter formstabile Elemente. Eine Formstabilität ist jedoch dann nicht gegeben, wenn die geometrische Ausgestaltung und/oder die geometrische Ausrichtung der Verpackungsfolie bereits durch die Schwerkraft veränderbar sind. Eine derartige Situation ergibt sich zum Beispiel bei einem Blatt Papier, dessen Ausrichtung durch die Schwerkraft in einfacher Weise verändert werden kann. Durch die Ausbildung der Verpackungsfolie in einer formstabilen Weise kann beispielsweise eine Schale durch die Verpackungsfolie ausgebildet werden, welche eine, insbesondere obige,

Öffnung aufweist, durch welche die Dentalvorrichtung in die Schale eingelegt werden kann. Sollte die Verpackungsfolie jedoch als eine forminstabile Verpackungsfolie ausgebildet sein, so kann diese insbesondere dazu dienen, eine Öffnung, insbesondere eine Öffnung einer Schale, abzudecken bzw. zu verschließen. Zweckmäßigerweise kann dabei die Festlegung einer derartigen forminstabilen Verpackungsfolie durch eine stoffschlüssige Festlegung erfolgen. Eine derartige stoffschlüssige Festlegung kann insbesondere ein Schweißen und/oder ein Kleben sein. Alternativ oder zusätzlich bevorzugt ist grundlegend auch ein Löten möglich. Durch diese stoffschlüssige Festlegung kann insbesondere eine sterile Umgebung weiter aufrechterhalten werden, denn durch die stoffschlüssige Fügung kann eine Dichtheit kostengünstig und sicher erreicht werden.

Vorteilhafterweise weist die Sterilgutverpackung, insbesondere die Verpackungsfolie, eine Öffnung bzw. einen Zugang auf, wobei die Öffnung bzw. der Zugang mit einer Abdeckfolie und/oder mit der oder einer Verpackungsfolie abgedeckt ist. In anderen Worten kann die Verpackungsfolie dazu ausgelegt sein, eine Schale mit einer Öffnung auszubilden, wobei diese Öffnung als ein Zugang angesehen werden kann, durch welchen ein Sterilgut, nämlich eine Dentalvorrichtung, in die Schale eingebracht werden kann. Diese Öffnung der Sterilgutverpackung kann insbesondere durch eine Abdeckfolie und/oder durch eine Verpackungsfolie abgedeckt sein. Hierdurch kann die Integritätsüberwachung der Sterilgutverpackung weiter gesteigert werden. Vorteilhafterweise ist, wie bereits ausgeführt, dabei die Abdeckfolie bzw. die Verpackungsfolie stoffschlüssig an den Bestandteilen fixiert, welche die Öffnung ausbilden, wobei diese Bestandteile insbesondere auch durch eine Verpackungsfolie wie vorgehend oder nachfolgend beschrieben ausgebildet sein können. Durch eine derartige Ausgestaltung der Sterilgutverpackung kann eine besonders einfache Ausgestaltung des Innenraums erreicht werden, welcher dazu dient, das Sterilgut aufzunehmen.

Ein weiterer Aspekt der Erfindung kann die Verwendung einer Sterilgutverpackung, in Form einer Dentalvorrichtungsverpackung, insbesondere wie vorgehend und nachfolgend beschrieben, zur Verpackung eines Sterilguts, nämlich einer Dentalvorrichtung, betreffen. Hierdurch können die bereits vorgehend und nachfolgend beschriebenen Vorteile und/oder Merkmale in einfacher Weise zur Verpackung eines Sterilguts erreicht werden und/oder verwendet werden.

Ein weiterer Aspekt der Erfindung kann ein Verfahren zur Herstellung einer Sterilgutverpackung, nämlich einer Dentalverpackungsvorrichtung, vorteilhafterweise wie vorgehend und nachfolgend beschrieben, betreffen. Zweckmäßigerweise umfasst ein derartiges Verfahren die Schritte:
- Bereitstellen einer Trennschicht;
- Aufbringen einer ersten Leitschicht auf einer ersten Fläche der Trennschicht, insbesondere mittels Aufdampfen;
- und/oder Aufbringen einer zweiten Leitschicht auf einer der ersten Fläche gegenüberlegenen Fläche der Trennschicht, insbesondere mittels Auf dampfen.

Durch ein derartiges Verfahren kann in einfacher und schneller sowie kostengünstiger Weise eine Sterilgutverpackung wie vorgehend und nachfolgend erstellt werden bzw. hergestellt werden. Zweckmäßigerweise kann das Verfahren dabei auch die bereits vorgehend und nachfolgend beschriebenen Vorrichtungsmerkmale in äquivalenter Weise für ein Verfahren beinhalten.

Ein weiterer Aspekt der Erfindung kann ein Verpackungssystem betreffen. Vorteilhafterweise umfasst ein derartiges Verpackungssystem eine Dentalvorrichtung und eine Sterilgutverpackung, insbesondere wie vorgehend und nachfolgend beschrieben. Durch eine derartige Ausgestaltung können in einem Verpackungssystem die bereits beschriebenen Vorteile, Merkmale und Ausgestaltung und die nachfolgend beschriebenen Vorteile, Merkmale und Ausgestaltungen erreicht werden.

In einer bevorzugten Ausführungsform weist das Verpackungssystem eine Dentalvorrichtung auf, welche eine Hauptdimension im Bereich zwischen 2-70 mm aufweist. Die Hauptdimension der Dentalvorrichtung ist dabei insbesondere diejenige Dimension, in welche sich die Länge der Dentalvorrichtung bestimmt und/oder in welche die Dentalvorrichtung seine größte Dimensionierung aufweist. Durch die Ausgestaltung dahingehend, dass die in der Dentalvorrichtungsverpackung aufgenommenen Dentalvorrichtungen eine Hauptdimension im Bereich von 2-70 mm aufweisen, kann die Dentalvorrichtung bzw. die Dentalvorrichtungsverpackung, welche die Dentalvorrichtung vollständig in ihrem Innenraum aufnimmt, für eine Vielzahl von Dentalvorrichtungen eingesetzt werden.

Weitere Vorteile und Merkmale der vorliegenden Erfindung ergeben sich aus der nachfolgenden Beschreibung mit Bezug auf die Figuren. Einzelne Merkmale der dargestellten Ausführungsformen können auch in anderen Ausführungsformen eingesetzt werden, sofern dies nicht ausdrücklich ausgeschlossen wurde. Es zeigen:
- Figur 1: eine Seitenansicht einer Verpackungsfolie; und
- Figur 2: einen Schnitt durch eine Dentalvorrichtungsverpackung.

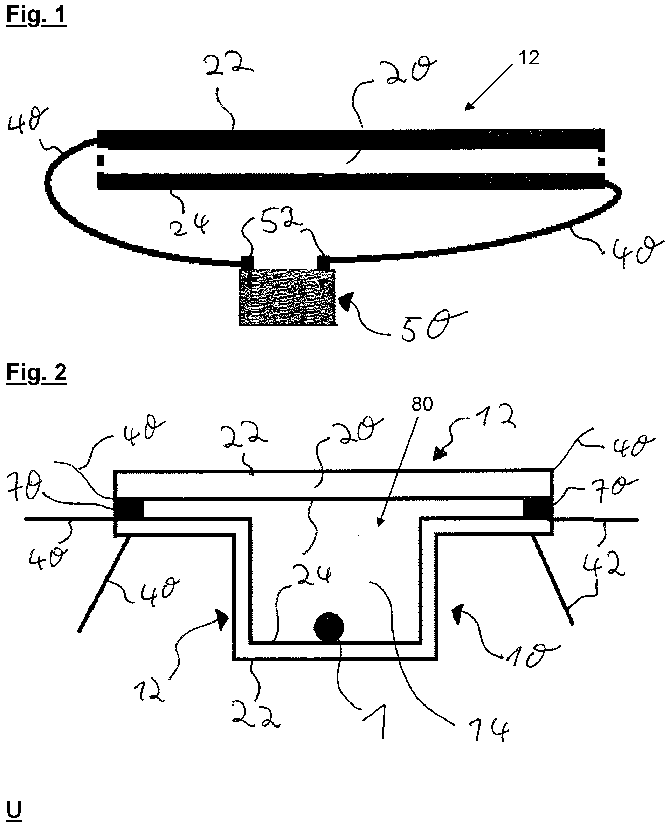

In **Figur 1** ist eine Verpackungsfolie 12 gezeigt, wobei die Verpackungsfolie 12 zumindest eine erste Leitschicht 22, eine Trennschicht 20 und eine zweite Leitschicht 24 aufweist. In Dickenrichtung zwischen der ersten Leitschicht, 22 und der zweiten Leitschicht 24 befindet sich dabei die Trennschicht 20. Die Trennschicht 20 ist sowohl bakterienundurchlässig als auch ein Isolator, sodass die Trennschicht 20 nicht elektrisch leitend ist. Die erste Leitschicht 22 als auch die zweite Leitschicht 24 verfügen jeweils über einen Primäranschluss 40. Der Primäranschluss 40 der ersten Leitschicht 22 als auch der Primäranschluss 40 der zweiten Leitschicht 24 sind jeweils mit unterschiedlichen Polen 52 der elektrischen Energiequelle 50 verbunden. Sollte daher die Integrität der Trennschicht 20 verloren gehen, so kann von dem ersten Pol 52 bzw. dem Pluspol 52 ein Strom über dem Primäranschluss 40 in die erste Leitschicht 22 gelangen und von diesem durch die Trennschicht bzw. die Integrationsverluststelle der Trennschicht 20 in die zweite Leitschicht 24 gelangen und von dieser über den Primäranschluss 40 der zweiten Leitschicht 24 zum anderen Pol 52 der elektrischen Energiequelle 50. Durch dieses Schließen des Stromkreises durch den Integritätsverlust der Trennschicht 20 kann in besonders einfacher Weise ein Integritätsverlust der Verpackungsfolie 12 festgestellt werden.

In **Figur 2** ist eine Dentalvorrichtungsverpackung 10 gezeigt. In dem Innenraum 14 der Dentalvorrichtungsverpackung 10 befindet sich eine Dentalvorrichtung 1. Die Dentalvorrichtungsverpackung 10 umfasst zwei unterschiedlich ausgebildete Verpackungsfolien 12, wobei jede dieser Verpackungsfolien 12 eine erste Leitschicht 22, eine Trennschicht 20, als auch eine zweite Leitschicht 24 aufweist. Prinzipiell kann dabei jede dieser Verpackungsfolien 12 der in der Figur 1 dargestellten Verpackungsfolien 12 entsprechen. Die untere Verpackungsfolie 12 ist dabei formstabil ausgebildet und verfügt über eine Schale, welche an ihrem oberen Ende eine Öffnung 80 ausbildet. Diese Öffnung 80 ist durch die weitere obere Verpackungsfolie 12 abgedeckt, wobei die beiden Verpackungsfolien 12 über eine stoffschlüssige Verbindung 70 miteinander verbunden sind. Die obere Verpackungsfolie 12 ist dabei derart ausgestaltet, dass die erste Leitschicht 22 und die zweite Leitschicht 24 jeweils einen Primäranschluss 40 aufweisen. Auch die untere Verpackungsfolie 12, welche formstabil ausgebildet ist, verfügt über eine erste Leitschicht 22 und über eine zweite Leitschicht 24, wobei diese jeweils einen Primäranschluss 40 als auch einen Sekundäranschluss 42 aufweisen.

### Bezugszeichen

- 1: - Dentalvorrichtung
- 10: - Dentalvorrichtungsverpackung
- 12: - Verpackungsfolie
- 14: - Innenraum
- 20: - Trennschicht
- 22: - erste Leitschicht
- 24: - zweite Leitschicht
- 40: - Primäranschluss
- 42: - Sekundäranschluss
- 50: - elektrische Energiequelle
- 52: - Pol der elektrischen Energiequelle
- 70: - stoffschlüssige Verbindung
- 80: - Öffnung
- U: - Umgebung

## Patentansprüche

1. Sterilgutverpackung, welche eine Dentalvorrichtungsverpackung (10) ist, umfassend eine Verpackungsfolie (12),
wobei die Dentalvorrichtungsverpackung (10), einen Innenraum (14) aufweist,
wobei die Verpackungsfolie (12) den Innenraum (14) der Dentalvorrichtungsverpackung (10), gegenüber der Umgebung (U) zumindest teilweise abgrenzt,
wobei die Verpackungsfolie (12) eine Trennschicht (20) aufweist,
wobei die Verpackungsfolie (12) eine erste Leitschicht (22) und eine zweite Leitschicht (24) aufweist,
wobei die erste und die zweite Leitschicht (22, 24) jeweils elektrisch leitend sind und jeweils einen Primäranschluss (40) aufweisen,
wobei die Trennschicht (20) zwischen der ersten Leitschicht (22) und der zweiten Leitschicht (24) angeordnet ist.

2. Sterilgutverpackung gemäß Anspruch 1,
wobei die erste und/oder die zweite Leitschicht (22, 24) flächig die Trennschicht (20) bedecken.

3. Sterilgutverpackung gemäß einem der vorhergehenden Ansprüche,
wobei die Trennschicht (20) nicht elektrisch leitend ist.

4. Sterilgutverpackung gemäß einem der vorhergehenden Ansprüche,
wobei die Trennschicht (20) bakterienundurchlässig ist.

5. Sterilgutverpackung gemäß einem der vorhergehenden Ansprüche,
wobei die Trennschicht (20) aus Kunststoff ist, insbesondere aus PET, PETG und/oder PE-HD.

6. Sterilgutverpackung gemäß einem der vorhergehenden Ansprüche,
wobei der Primäranschluss (40) der ersten Leitschicht (22) mit einem Pol (52) einer elektrischen Energiequelle (50) verbunden ist oder verbindbar ist, und
wobei der Primäranschluss (40) der zweiten Leitschicht (24) mit dem anderen Pol (52) der elektrischen Energiequelle (50) verbunden ist oder verbindbar ist.

7. Sterilgutverpackung gemäß einem der vorhergehenden Ansprüche,
wobei die elektrische Energiequelle (50) eine Wechselstromquelle oder eine Gleichstromquelle ist.

8. Sterilgutverpackung gemäß einem der vorhergehenden Ansprüche,
wobei die erste Leitschicht (22) und/oder die zweite Leitschicht (24) einen Sekundäranschluss (42) aufweisen oder mit einem Sekundäranschluss (42) verbunden sind.

9. Sterilgutverpackung gemäß einem der vorhergehenden Ansprüche,
wobei die Trennschicht (20), die erste Leitschicht (22) und/oder die zweite Leitschicht (24) gasdurchlässig und/oder gammastrahlungsdurchlässig ist.

10. Sterilgutverpackung gemäß einem der vorhergehenden Ansprüche,
wobei die erste und/oder die zweite Leitschicht (22, 24) zumindest teilweise, bevorzugt vollständig, aus einem Material ausgebildet ist oder beschichtet ist, welches seine Farbe bei einem durch die erste und/oder die zweite Leitschicht (22, 24) strömenden elektrischen Strom ändert.

11. Sterilgutverpackung gemäß einem der vorhergehenden Ansprüche,
wobei das Verhältnis der Dicke der ersten Leitschicht bzw. der Dicke der zweiten Leitschicht zu der Dicke der Trennschicht (20) in einem Bereich von 0,25 bis 1,5, bevorzugt in einem Bereich von 0,4 bis 1 und besonders bevorzugt in einem Bereich von 0,45 bis 0,55 liegt.

12. Sterilgutverpackung gemäß einem der vorhergehenden Ansprüche,
wobei die Verpackungsfolie (12) formstabil oder form instabil ist.

13. Verwendung einer Sterilgutverpackung gemäß einem der vorgehenden Ansprüche zur Verpackung von Dentalvorrichtungen.

14. Verfahren zur Herstellung einer Sterilgutverpackung gemäß einem der Ansprüche 1 bis 12, umfassend die Schritte:
- Bereitstellen einer Trennschicht (20);
- Aufbringen einer ersten Leitschicht (22) auf einer ersten Fläche der Trennschicht (20), insbesondere mittels Aufdampfen;
- Aufbringen einer zweiten Leitschicht (22) auf einer der ersten Fläche gegenüberliegenden Fläche der Trennschicht (20), insbesondere mittels Aufdampfen;

15. Verpackungssystem umfassend ein Sterilgut, nämlich eine Dentalvorrichtung (1), und eine Sterilgutverpackung gemäß einem der vorhergehenden Ansprüche 1 bis 12.

## Claims

1. A sterile goods package which is a dental device package (10) comprising a packaging film (12),
wherein the dental device packaging (10) has an interior space (14),
wherein the packaging film (12) at least partially delimits the interior (14) of the dental device packaging (10) with respect to the environment (U),
wherein the packaging film (12) has a separating layer (20),
wherein the packaging film (12) has a first conductive layer (22) and a second conductive layer (24),
wherein the first and second conductive layers (22, 24) are each electrically conductive and each have a primary terminal (40),
wherein the separating layer (20) is arranged between the first conductive layer (22) and the second conductive layer (24).

2. Sterile goods packaging according to claim 1,
wherein the first and/or the second conductive layer (22, 24) cover the release layer (20) over a large area.

3. Sterile goods packaging according to one of the preceding claims,
wherein the separating layer (20) is not electrically conductive.

4. Sterile goods packaging according to one of the preceding claims,
wherein the separating layer (20) is impermeable to bacteria.

5. Sterile goods packaging according to one of the preceding claims,
wherein the separating layer (20) is made of plastic, in particular PET, PETG and/or PE-HD.

6. Sterile packaging according to any one of the preceding claims,
wherein the primary terminal (40) of the first conductive layer (22) is connected or connectable to one pole (52) of an electrical energy source (50), and
wherein the primary connection (40) of the second conductive layer (24) is connected or connectable to the other pole (52) of the electrical energy source (50).

7. Sterile goods packaging according to one of the preceding claims,
wherein the electrical energy source (50) is an alternating current source or a direct current source.

8. Sterile goods packaging according to one of the preceding claims,
wherein the first conductive layer (22) and/or the second conductive layer (24) comprise a secondary connection (42) or are connected to a secondary connection (42).

9. Sterile goods packaging according to one of the preceding claims,
wherein the separating layer (20), the first conductive layer (22) and/or the second conductive layer (24) is gas-permeable and/or gamma-radiation-permeable.

10. Sterile goods packaging according to one of the preceding claims,
wherein the first and/or the second conductive layer (22, 24) is at least partially, preferably completely, formed of or coated with a material which changes its color when an electric current flows through the first and/or the second conductive layer (22, 24).

11. Sterile goods packaging according to one of the preceding claims,
wherein the ratio of the thickness of the first conductive layer or the thickness of the second conductive layer to the thickness of the release layer (20) is in a range from 0.25 to 1.5, preferably in a range from 0.4 to 1 and particularly preferably in a range from 0.45 to 0.55.

12. Sterile goods packaging according to one of the preceding claims,
wherein the packaging film (12) is dimensionally stable or dimensionally unstable.

13. Use of a sterile packaging according to one of the preceding claims for packaging dental devices.

14. A method of making a sterile package according to any one of claims 1 to 12, comprising the steps of:
- Providing a release layer (20);
- applying a first conductive layer (22) to a first surface of the release layer (20), in particular by vapour deposition;
- applying a second conductive layer (22) to a surface of the release layer (20) opposite the first surface, in particular by vapour deposition;

15. A packaging system comprising a sterile product, namely a dental device (1), and a sterile product package according to any one of the preceding claims 1 to 12.

## Revendications

1. Emballage de produit stérile qui est un emballage de dispositif dentaire (10), comprenant un film d'emballage (12),
dans lequel
l'emballage de dispositif dentaire (10) présente un espace intérieur (14),
le film d'emballage (12) délimite au moins partiellement l'espace intérieur (14) de l'emballage de dispositif dentaire (10) par rapport à l'environnement (U),
le film d'emballage (12) présente une couche de séparation (20),
le film d'emballage (12) présente une première couche conductrice (22) et une deuxième couche conductrice (24),
les première et deuxième couches conductrices (22, 24) sont chacune électriquement conductrice et présentent chacune une connexion primaire (40),
la couche de séparation (20) est disposée entre la première couche conductrice (22) et la deuxième couche conductrice (24).

2. Emballage de produit stérile selon la revendication 1,
dans lequel la première et/ou la deuxième couche conductrice (22, 24) recouvre(nt) la couche de séparation (20) sur sa surface.

3. Emballage de produit stérile selon l'une des revendications précédentes, dans lequel la couche de séparation (20) n'est pas électriquement conductrice.

4. Emballage de produit stérile selon l'une des revendications précédentes, dans lequel la couche de séparation (20) est imperméable aux bactéries.

5. Emballage de produit stérile selon l'une des revendications précédentes, dans lequel la couche de séparation (20) est en matière plastique, en particulier en PET, PETG et/ou PE-HD.

6. Emballage de produit stérile selon l'une des revendications précédentes,
dans lequel la connexion primaire (40) de la première couche conductrice (22) est reliée ou peut être reliée à un pôle (52) d'une source d'énergie électrique (50), et
la connexion primaire (40) de la deuxième couche conductrice (24) est reliée ou peut être reliée à l'autre pôle (52) de la source d'énergie électrique (50).

7. Emballage de produit stérile selon l'une des revendications précédentes, dans lequel la source d'énergie électrique (50) est une source de courant alternatif ou une source de courant continu.

8. Emballage de produit stérile selon l'une des revendications précédentes, dans lequel la première couche conductrice (22) et/ou la deuxième couche conductrice (24) présentent une connexion secondaire (42) ou sont reliées à une connexion secondaire (42).

9. Emballage de produit stérile selon l'une des revendications précédentes, dans lequel la couche de séparation (20), la première couche conductrice (22) et/ou la deuxième couche conductrice (24) est perméable aux gaz et/ou perméable aux rayons gamma.

10. Emballage de produit stérile selon l'une des revendications précédentes, dans lequel la première et/ou la deuxième couche conductrice (22, 24) est réalisée ou revêtue au moins partiellement, de préférence entièrement, d'un matériau qui change de couleur lorsqu'un courant électrique circule à travers la première et/ou la deuxième couche conductrice (22, 24).

11. Emballage de produit stérile selon l'une des revendications précédentes, dans lequel le rapport entre l'épaisseur de la première couche conductrice ou l'épaisseur de la deuxième couche conductrice et l'épaisseur de la couche de séparation (20) est compris dans une plage allant de 0,25 à 1,5, de préférence dans une plage allant de 0,4 à 1 et de manière particulièrement préférée dans une plage allant de 0,45 à 0,55.

12. Emballage de produit stérile selon l'une des revendications précédentes, dans lequel le film d'emballage (12) est de forme stabile ou de forme instable.

13. Utilisation d'un emballage de produit stérile selon l'une des revendications précédentes pour l'emballage de dispositifs dentaires.

14. Procédé de fabrication d'un emballage de produit stérile selon l'une des revendications 1 à 12, comprenant les étapes consistant à :
- fournir une couche de séparation (20) ;
- appliquer une première couche conductrice (22) sur une première surface de la couche de séparation (20), en particulier par dépôt en phase vapeur ;
- appliquer une deuxième couche conductrice (22) sur une surface de la couche de séparation (20) opposée à la première surface, en particulier par dépôt en phase vapeur.

15. Système de conditionnement comprenant un produit stérile, à savoir un dispositif dentaire (1), et un emballage de produit stérile selon l'une des revendications précédentes 1 à 12.
